Europäisches Patentamt

**European Patent Office**

Office européen des brevets

(19)

(11) Numéro de publication: **0 221 813**
**B1**

(12) **FASCICULE DE BREVET EUROPEEN**

(45) Date de publication du fascicule du brevet:
**01.03.89**

(21) Numéro de dépôt: **86402317.1**

(22) Date de dépôt: **16.10.86**

(51) Int. Cl.⁴: **B01J 19/24, B01J 12/00,**
**B01J 4/00, C07C 29/15**
**// C01B3/34**

(54) Procédé, réacteur d'oxydation d'une charge oxydable en phase gazeuse et son utilisation.

(30) Priorité: **17.10.85 FR 8515550**
**30.12.85 FR 8519427**

(43) Date de publication de la demande:
**13.05.87 Bulletin 87/20**

(45) Mention de la délivrance du brevet:
**01.03.89 Bulletin 89/9**

(84) Etats contractants désignés:
**DE FR GB IT NL**

(56) Documents cités:
**DE-A- 1 804 093**
**FR-A- 1 395 256**
**US-A- 2 772 149**
**US-A- 4 012 201**
**US-A- 4 381 187**

(73) Titulaire: **INSTITUT FRANCAIS DU PETROLE, 4, Avenue de Bois-Préau, F-92502 Rueil-Malmaison(FR)**

(72) Inventeur: **Alagy, Jacques, 24, Chemin Beckensteiner, F-69260 Charbonnieres les Bains(FR)**
Inventeur: **Busson, Christian, Chemin de Cogny, F-69570 Dardilly(FR)**

(74) Mandataire: **Aubel, Pierre et al, Institut Français du Pétrole Département Brevets 4, avenue de Bois Préau, F-92502 Rueil-Malmaison(FR)**

**Description**

La présente invention concerne un nouveau réacteur, son utilisation et un procédé d'oxydation d'une charge oxydable en phase gazeuse par un mélange de gaz contenant au moins un gaz oxydant.

Elle s'applique plus spécialement à l'oxydation lente et partielle de charges oxydables telles que par exemple des hydrocarbures en vue de la préparation de gaz de synthèse comprenant essentiellement du monoxyde de carbone et de l'hydrogène, par exemple pour la synthèse de méthanol et d'alcools homologues supérieurs. Elle peut s'appliquer aussi par exemple, à l'oxydation des effluents de réformage à la vapeur, du benzène, ou aux réactions d'ammonoxydation.

Bien que les gaz oxydants puissent être notamment l'oxygène, l'ozone ou les halogènes, on ne considérera à titre d'exemple, que les réactions avec l'oxygène.

Il est connu de réaliser une oxydation partielle du méthane, comme indiqué, par exemple dans le brevet US-A 2 621 117.

La réaction se fait dans une flamme où le mélange des gaz n'est jamais parfait. Dans ces conditions, on atteint rapidement des températures élevées dans les zones riches en oxygène.

Les gaz produits à haute température sont ensuite mélangés dans une zone riche en charge à oxyder et provoquent le craquage des molécules avec formation de carbone susceptible par exemple d'encrasser les catalyseurs dans la suite du procédé et donc de diminuer le rendement de la réaction.

Dans le cas du méthane, on observe une production de carbone et les gaz de synthèse doivent être ultérieurement dépoussiérés avant leur utilisation, par exemple pour la synthèse du méthanol à partir de l'oxyde de carbone et de l'hydrogène.

Outre la formation de noir de carbone, il peut se produire une surchauffe excessive de la zone où s'effectue le contact des gaz réactionnels et dans un grand nombre de cas, ces effets indésirables sont essentiellement attribuables au dispositif de mélange des gaz réactionnels à l'entrée du réacteur, le mélange des gaz étant effectué à une vitesse trop lente par rapport à la vitesse de réaction en phase gazeuse.

C'est le cas lorsque l'oxygène est injecté à travers un seul canal qui doit d'ailleurs avoir une section suffisante pour admettre la totalité du débit et bien que le gaz soit injecté à grande vitesse à travers cette section, la vitesse de dispersion des molécules d'oxygène est lente, comparée à celle de la réaction.

De plus, le jet d'oxygène à l'endroit où il sort de son orifice, est généralement dans l'environnement du gaz à oxyder qui circule à faible vitesse dans le réacteur, ce qui n'est pas favorable à une dispersion rapide des molécules d'oxygène.

L'art antérieur est notamment illustré par les brevets DE-A 1 804 093 et FR-A 1 395 256.

Selon le brevet US-A 2 772 149, le mélange de gaz réactionnels se fait à la surface d'un diaphragme poreux de faible surface, où oxygène et hydrocarbures sont injectés dans la même direction; cela présente l'avantage de mélanger rapidement les gaz réactionnels.

Néanmoins, à cause de la vitesse lente du gaz traversant les pores du diaphragme, la réaction a lieu essentiellement à la surface de sortie dudit diaphragme, lequel doit en conséquence être conçu pour résister aux hautes températures. Dans les unités de grande capacité, ce dispositif nécessiterait une grande surface pour le diaphragme, ce qui le rend onéreux et impraticable.

Le brevet EP-B 0 001 946 décrit un réacteur où l'oxygène, étant donné son débit important, est injecté dans le gaz de procédé par l'intermédiaire d'une multitude de canaux parallèles se terminant chacun par un orifice de sortie dont l'une au moins des dimensions est très réduite, tel qu'une fente dont la largeur est de préférence inférieure à 8 mm.

De plus, afin d'augmenter la vitesse de dispersion de l'oxygène dans le gaz de procédé, celui-ci est animé d'un violent mouvement hélicoïdal autour desdits canaux, obtenu par une injection tangentielle de ce gaz sur les parois intérieures de l'appareil.

Par ailleurs, il est bien connu, notamment par le livre de G. de Soete et A. Feugier: "Aspects physiques et chimiques de la combustion" Editions Technip, pages 87 à 93, d'utiliser l'effet de paroi pour diminuer la vitesse réactionnelle et éviter la propagation de la flamme.

Dans le cas présent, la présence d'oxygène pur et de température élevée impliquant un flux thermique élevé nécessitent des dispositifs d'arrêt de flamme permettant à la réaction de se poursuivre sans explosion, bien que l'on soit à l'intérieur des limites explosives (notamment dans le cas de l'oxydation partielle du méthane).

Les objectifs que l'on se propose d'atteindre et qui répondent aux problèmes soulevés par l'art antérieur sont essentiellement les suivants:

– une distribution en oxygène et en charge adaptée à la recherche d'un mélange quasi homogène, parfaitement contrôlée, entre l'oxygène et la charge à oxyder. Cette distribution doit être particulièrement adaptée à la dispersion rapide des molécules d'oxygène.

– un "arrêt ou coincement de flamme", évitant l'explosion et permettant cependant d'opérer à des températures pouvant atteindre plus de 1.000°C, avec le souci de protéger le réacteur et le dispositif mélangeur de la chaleur excessive dégagée lors de l'oxydation partielle.

La présente invention propose un nouveau procédé remédiant aux inconvénients de l'art antérieur. Elle concerne plus précisément un procédé d'oxydation d'une charge oxydable en phase gazeuse par un mélange de gaz contenant au moins un gaz oxydant dans lequel on recueille des produits réactionnels.

De manière plus précise, on met en contact le mélange de gaz et la charge oxydable dans une zone de mise en contact et de mélange située entre au moins une première zone parcourue par ladite charge et au moins une seconde zone parcourue par les produits réactionnels d'oxydation ainsi obtenus, lesdites première et seconde zones définissant une multiplicité d'espaces multidirectionnels présentant des passages ayant, suivant au moins une direction, une dimension au plus égale à 10 millimètres, cette dimension correspondant à la distance de coincement de la flamme pouvant résulter de l'oxydation de ladite charge, ladite zone de mise en contact comprenant une zone d'alimentation en mélange oxydant qui comporte une pluralité de conduits sensiblement parallèles à parois poreuses et qui est située à une distance de la première zone et de la seconde zone au plus égale à la distance de coincement.

Avantageusement, cette multiplicité d'espaces dans la première et la seconde zone est située au voisinage immédiat de la zone de mise en contact.

L'invention concerne aussi un réacteur pour la mise en oeuvre du procédé.

Ce réacteur comprend des moyens d'alimentation en gaz oxydant et en charge oxydable et des moyens d'évacuation des produits réactionnels. Il comporte en outre, en combinaison:
– au moins un distributeur en gaz oxydant relié d'une part aux moyens d'alimentation en gaz oxydant et d'autre part à une pluralité de conduits parallèles à parois poreuses en matière céramique,
– au moins une première capacité comportant un garnissage en matière céramique qui définit une multiplicité d'espaces multidirectionnels présentant des passages ayant, suivant au moins une direction, une dimension au plus égale à 10 mm, cette dimension correspondant à la distance de coincement de la flamme pouvant résulter de l'oxydation de ladite charge, ladite première capacité étant reliée auxdits moyens d'alimentation en charge oxydable, et
– au moins une seconde capacité comportant un garnissage en matière céramique qui définit une multiplicité d'espaces multidirectionnels présentant des passages ayant, suivant au moins une direction, une dimension au plus égale à 10 mm, cette dimension correspondant à la distance de coincement de la flamme pouvant résulter de l'oxydation de ladite charge, ladite seconde capacité étant reliée auxdits moyens d'évacuation des produits réactionnels, ladite première et ladite seconde capacités étant situées de part et d'autre desdits conduits poreux à une distance au plus égale à la distance de coincement.

Avantageusement, la distance est comprise entre 0,05 et 10 mm; de préférence, elle est comprise entre 0,1 et 5 mm.

Les conduits sont avantageusement poreux sur toute leur surface.

Les espaces relatifs à la première et à la seconde capacités ont une dimension avantageusement comprise entre 0,1 et 5 mm.

De préférence, l'espace ainsi aménagé est uniforme.

Compte tenu des niveaux thermiques atteints (1.400°C par exemple pour l'oxydation partielle du méthane) et de la présence d'oxygène avantageusement pur, on fait s'écouler ladite charge et les produits réactionnels dans des espaces entourés de parois en matière céramique et ledit mélange oxydant dans des canaux dont les parois sont en matière céramique.

On peut utiliser comme matériaux réfractaires des céramiques tels que la mullite, le carbure de silicium, la cordiérite, l'alumine la silice, les nitrures de silicium comme par exemple $Si_3N_4$, les oxydes d'alcalino-terreux, les oxydes de métaux de transition et leurs mélanges.

De préférence, le carbure de silicium est choisi, car il présente une bonne conductibilité thermique et favorise ainsi une meilleure uniformité de température. De plus, ce matériau étant facilement extrudable, la mise en œuvre de tels ensembles est rendue plus aisée.

Chaque capacité (la première ou la seconde) présente au voisinage des conduits à parois poreuses, une surface d'écoulement sensiblement égale à celle occupée par ces conduits. De préférence, les surfaces d'écoulement de la charge d'une part, et des produits réactionnels d'autre part, sont sensiblement vis-à-vis de la surface réellement occupée par les conduits poreux de sorte qu'il y ait correspondance des flux de gaz.

Ainsi, par exemple, au niveau de la zone du distributeur en gaz oxydant qui n'est pas poreuse, font face des zones correspondant à la première et à la seconde capacité bouchées par une pâte céramique cuite et dans lesquelles il n'y a pas d'écoulement.

Le distributeur comporte un conduit d'amenée sur lesquels sont fixés plusieurs conduits poreux couvrant entièrement, selon un mode de réalisation, une partie au moins de la section du réacteur qui fait face aux capacités et à leur garnissage. On a obtenu de bons résultats lorsque la section couverte par ces conduits poreux est sensiblement perpendiculaire à l'axe et du réacteur, et donc à la direction de l'écoulement de la charge. Cependant, la section couverte par les conduits, au lieu d'être droite, peut être sensiblement oblique et donc inclinée par rapport au plan sensiblement perpendiculaire à l'axe du réacteur.

Les conduits poreux peuvent être disposés en couches décalées non adjacentes de part et d'autre du distributeur sur un même plan, pour faciliter l'écoulement des flux.

Dans le cas particulièrement avantageux où le réacteur comporte deux distributeurs en gaz oxydant, chacun d'eux est relié à une pluralité de conduits à parois poreuses sensiblement parallèles et définissent ainsi une première et une seconde couche de conduits qui sont décalées sans se toucher l'une par rapport à l'autre. Cette disposition facilite un meilleur contrôle, notamment thermique, de la réaction tout en permettant l'écoulement des flux. L'écartement entre deux conduits dans un même plan radial d'une même couche est au plus égal à la largeur d'un conduit.

Lorsque les conduits de mélange oxydant, par exemple d'oxygène sont adjacents, l'un au moins d'entre eux peut être percé d'une pluralité de trous de part et d'autre d'un plan longitudinal passant par son axe, ces trous ayant une dimension comprise entre 0,05 et 10 mm (distance de coincement) de façon à permettre l'écoulement des flux et le "coincement" de la flamme.

Dans le cas où l'on a affaire à un seul distributeur, le bord supérieur des conduits de la rangée inférieure est plus haut que le bord inférieur des conduits de la rangée supérieure.

La largeur de chaque conduit poreux est variable suivant la taille du réacteur. Elle est comprise par exemple entre environ 5 et 50 mm, de préférence entre environ 10 et 20 mm.

La longueur de chaque conduit poreux est choisie, suivant un mode de réalisation, de telle façon que la section totale du réacteur soit entièrement couverte par les conduits, quelle que soit la géométrie de ce réacteur. Dans le cas d'un réacteur de forme sensiblement cylindrique et dont la section en coupe a une forme circulaire, la longueur des conduits sensiblement parallèles à paroi poreuse s'inscrit dans un cercle coaxial au cercle défini par la section circulaire de la paroi interne du réacteur.

Cependant, si les conduits sont dans un autre plan présentant une inclinaison par rapport au plan sensiblement perpendiculaire à l'axe du réacteur, la longueur de ces conduits s'inscrit dans l'ellipse définie par la section du réacteur cylindrique par le plan des conduits.

On crée ainsi une nappe très uniforme d'oxygène sur toute la section utile du réacteur, cette nappe étant balayée par la charge à oxyder elle-même répartie uniformément comme décrit plus loin, ce qui favorise un micromélange dans la zone confinée entre les capacités et les conduits d'oxygène et une dispersion rapide.

La porosité est choisie de telle sorte que pour un débit donné la perte de charge sera suffisante pour que tous les conduits poreux soient alimentés en oxygène à partir du distributeur d'amenée.

On choisira, de préférence, sans que cela soit limitatif, une perte de charge comprise entre 0,05 et 10 bars.

Le garnissage peut comprendre au moins un monolithe remplissant chacun des capacités. Ce monolithe comporte une pluralité de canaux juxtaposés, de préférence sensiblement parallèles entre eux et à l'axe du réacteur, et de section comprise entre 0,0025 et 100 mm² et de préférence comprise entre 0,1 et 20 mm². De façon avantageuse, la surface de chaque canal est sensiblement égale.

Cette solution présente notamment l'avantage de répartir la charge de manière homogène selon l'axe du monolithe.

Le garnissage peut comprendre, selon un autre mode de réalisation, des éléments particulaires en céramique, par exemple sous forme de billes et de bâtonnets, de taille comprise entre 0,01 et 10 mm et de préférence entre 0,1 et 5 mm.

De préférence, on utilisera des billes qui, mises en contact, offrent un espace maximal de longueur au plus égale à leur rayon, ce qui permet de choisir la taille de ces billes en fonction de la distance de coincement voulue.

Dans ces conditions, les espaces définis par les éléments particulaires sont notamment d'autant plus faibles que les éléments particulaires ont par exemple une taille de petite dimension. On peut alors procéder à des réactions d'oxydation sous pression et stœchiométriques en présence d'oxygène sensiblement pur, qui nécessitent des distances de coincement de flamme très faibles, que l'on peut difficilement atteindre par la technologie du monolithe.

En effet, l'élaboration d'un monolithe de section unitaire très faible est difficilement réalisable et de manipulation fragile lors du chargement et du déchargement.

Par ailleurs, la présence de garnissage sous forme d'éléments particulaires facilite les opérations de charge et de décharge dans les capacités, ainsi que la régénération de ces particules par calcination du carbone déposé ou par voie chimique (acide par exemple) surtout si l'on traite des charges lourdes contenant des métaux tels que le nickel et le vanadium.

Selon un autre mode de réalisation, l'une au moins des capacités renferme un garnissage comprenant au moins un monolithe qui comporte une pluralité de canaux juxtaposés, de préférence sensiblement parallèles entre eux et à l'axe du réacteur. Ces canaux peuvent avoir généralement une surface sensiblement comprise entre 0,025 et 100 mm², mais ils peuvent avoir aussi une surface plus grande pouvant atteindre par exemple 25 cm², ce qui permet de compartimenter l'une au moins des capacités.

En outre, dans les deux cas ci-dessus, au moins un de ces canaux comporte au moins en partie un remplissage sous forme d'éléments particulaires en céramique de taille comprise entre 0,01 et 10 mm et avantageusement comprise entre 0,1 et 5 mm. De cette façon, la charge oxydable est bien canalisée, ce qui permet une distribution sensiblement uniforme de cette charge au niveau du distributeur en gaz oxydant et un débit régulier sur la totalité de la section du réacteur car il n'y a pas, dans ce cas, possibilité de courants préférentiels. De manière générale, les éléments particulaires peuvent comprendre un catalyseur.

Les particules peuvent être de n'importe quelle forme, régulière ou irrégulière. Seuls, importent les espaces définis entre leurs parois et c'est en fonction de ce paramètre qu'est choisie la taille des particules et notamment le diamètre des billes.

Par exemple, la longueur de la première et de la seconde capacité ou de chaque canal unitaire, lorsqu'on a affaire à un monolithe, est de 10 mm à 3.000 mm. De préférence, la longueur de la capacité en amont de la zone de contact avec l'oxygène peut être inférieure à celle de la capacité en aval et égale de 20 mm à 500 mm pour la première et de 100 mm à 2.000 mm pour la seconde.

Avant d'arriver au niveau du distributeur d'oxygène, la charge à oxyder, à l'état gazeux, est canalisée et s'écoule sensiblement selon l'axe du réacteur grâce au garnissage de la première capacité dont une extrémité fait face au distributeur.

La section des espaces (par exemple des canaux dans le cas des monolithes) et conduits a au choix l'une au moins des formes suivantes qui ne sont pas limitatives: polygonale, carrée, rectangulaire, cylindrique, elliptique, circulaire ou triangulaire. Dans le cas des monolithes, la section des canaux unitaires est avantageusement carrée.

La charge gazeuse circule sensiblement par exemple de bas en haut ou de haut en bas dans le cas d'un réacteur vertical et entre en contact dans la zone confinée entre le garnissage et les conduits poreux, avec l'oxygène.

A cet effet, le ou les monolithes en amont (premier) et en aval (second) du distributeur d'oxygène sont creusés de telle sorte qu'un espace de préférence uniforme est aménagé entre les conduits poreux et les premier et second monolithes, de profondeur au plus égale à 10 mm (distance de coincement de flamme) et de préférence de 0,1 à 5 mm, cet espace définissant un ensemble de cavités sensiblement parallèles entre elles aux conduits poreux.

La charge est ainsi introduite le plus près possible des conduits poreux distributeurs d'oxygène, dans la mesure où l'extrémité ouverte des monolithes épouse la forme de ces conduits. Il en est de même avec les particules qui peuvent même être en contact direct avec les conduits d'oxygène, pour autant que, par exemple, le diamètre des billes soit compatible avec la distance de coincement de flamme.

Grâce au dispositif, la charge à oxyder est bien distribuée de manière uniforme sur toute la section de la capacité au niveau du distributeur d'oxygène sans qu'il y ait de rétromélange. D'autre part, en raison de la faible section des espaces, on évite l'explosion (ou retour de flamme) grâce au phénomène dit de coincement de flamme.

Après un mélange homogène avec l'oxygène, les produits de réaction sont à nouveau canalisés à l'aide d'au moins une seconde capacité comportant une multiplicité d'espaces de forme et de section définie ci-dessus.

Les charges à traiter ont un temps de résidence dans la zone réactionnelle compris entre 2 et 10.000 ms et plus avantageusement compris entre 50 et 1.000 ms.

L'invention sera mieux comprise par la description de quelques modes de réalisation, donnés à titre illustratif mais nullement limitatif qui en sera faite ci-après à l'aide des figures annexées:

– la figure 1 représente le réacteur selon l'invention, suivant une coupe axiale,

– les figures 2 et 2A montrent une coupe transversale de l'alimentation en oxygène avec un distributeur (Fig. 2) ou deux (Fig. 2A),

– les figures 3 et 3A illustrent une vue de dessus de cette alimentation,

– les figures 4A, 4B et 4C représentent des modes d'arrivée de l'oxygène,

– les figures 5 et 5A montrent une structure de monolithe en nid d'abeille,

– les figures 6 et 6A représentent une vue détaillée de l'alimentation en oxygène (simple ou double) au voisinage des monolithes amont et aval, et

– la figure 7 illustre un autre mode de réalisation du réacteur à capacités comprenant un garnissage d'éléments particuliers.

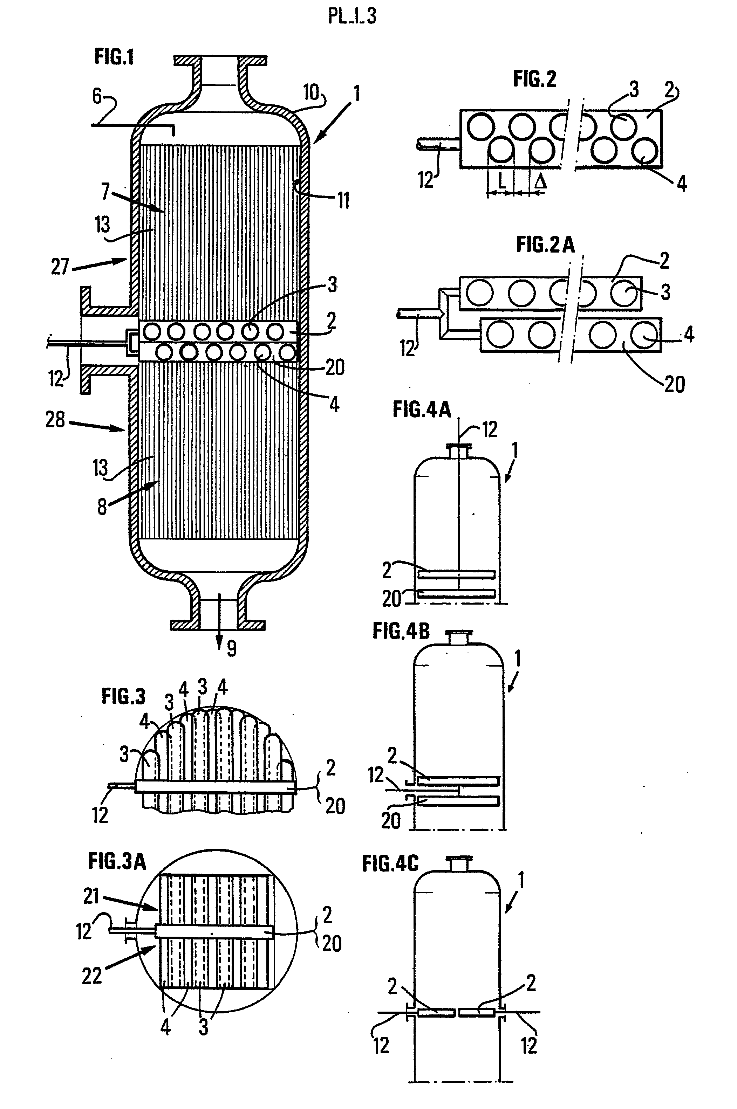

Sur la figure 1, on a représenté, selon un mode de réalisation, un réacteur 1 vertical cylindrique de forme allongée comprenant deux·distributeurs en oxygène 2 et 20, chacun d'eux étant relié à une couche constituée par une pluralité de conduits 3 et 4 en mullite, parallèles et poreux sur sensiblement toute leur surface, chacun des conduits ayant une forme, par exemple ronde de 1 cm de diamètre.

La surface poreuse, vue de dessus, est sensiblement celle d'une figure géométrique carrée (Fig. 3A) séparée en deux demi-rectangles sensiblement égaux 21 et 22 par le distributeur en oxygène 2.

La surface poreuse ainsi offerte à la charge est environ de 100 $cm^2$ et la porosité est choisie de telle façon que la perte de charge soit sensiblement égale à 0,5 bar.

Le distributeur et les conduits poreux ainsi alimentés par une ligne 12 sont disposés sensiblement perpendiculairement à l'axe du réacteur dans une zone sensiblement en amont par rapport au plan radial du réacteur. Les deux couches 3 et 4 sont alternées et non adjacentes de part et d'autre du distributeur comme le montrent les figures 2 et 2A.

L'écartement Δ entre deux conduits poreux d'une même couche permettant l'écoulement des flux est au plus égal à la largeur L des conduits, soit 1 cm et la distance entre les deux couches est voisine de 6 cm.

Selon la figure 3 illustrant un autre mode de réalisation, les conduits poreux couvrent sensiblement la totalité de la section du réacteur par alternance de couches décalées de conduits non adjacents 3 et 4 disposés de part et d'autre du distributeur d'oxygène 2.

La longueur de chaque conduit est choisie de telle façon que son extrémité est sensiblement tangente au cercle correspondant à la section du réacteur, cercle dont le diamètre est sensiblement égal à celui de l'intérieur du réacteur dans lequel est installé le distributeur.

L'alimentation en oxygène peut s'effectuer par l'intermédiaire d'une ligne 12 reliée aux distributeurs 2 et 20 par exemple, suivant l'axe du réacteur (Fig. 4A), ou bien latéralement (Fig. 4B), ou encore avec deux demi-distributeurs (Fig. 4C), sans que ces modes de réalisation soient limitatifs.

La charge oxydable gazeuse préalablement préchauffée vers 450°C arrive par une conduite 6 et alimente de haut en bas une première capacité 27 comprenant un premier monolithe 7 de longueur approximative égale à 20 cm qui comporte une pluralité de canaux 13 en mullite juxtaposés sensiblement parallèles entre eux et à l'axe du réacteur.

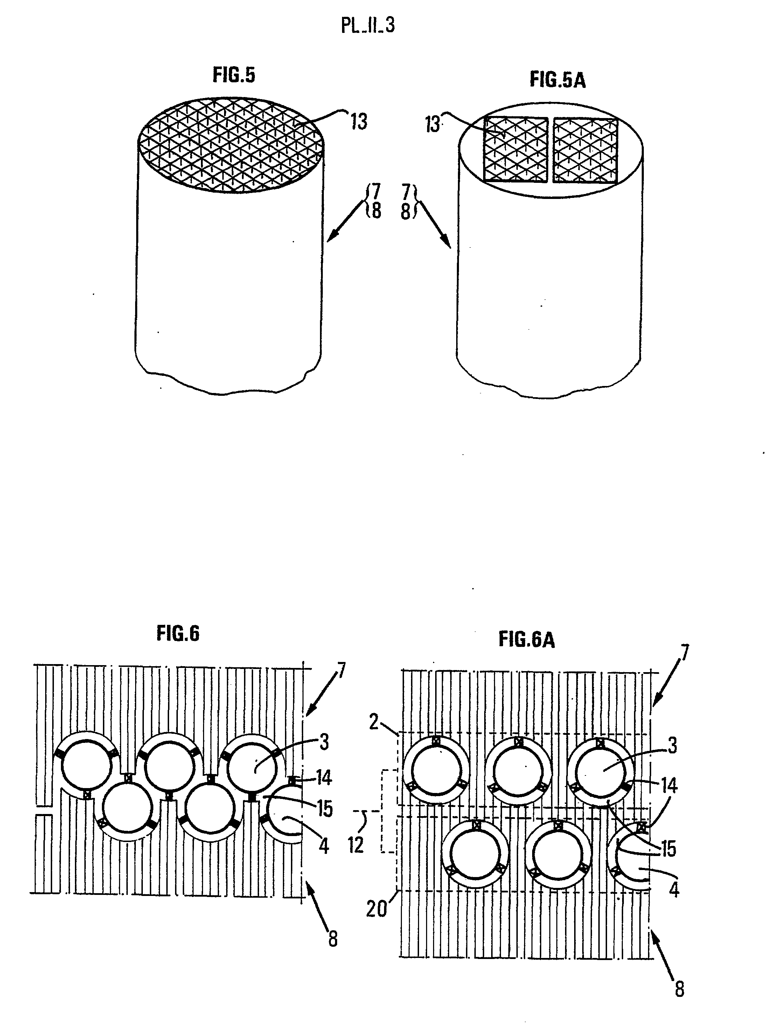

Cette structure est semblable à celle d'un nid d'abeille (Fig. 5) dont l'ensemble de la section serait circulaire.

Dans le cas présent, l'ensemble de la surface par laquelle s'écoule la charge correspond sensiblement à la surface poreuse de laquelle s'échappe l'oxygène. A cet effet, comme le montre la figure 5A, une pâte céramique cuite bouche les parties du monolithe ne faisant pas face à la zone de distribution de l'oxygène, par exemple la zone centrale du distributeur 2 ou 20. Par conséquent, la surface réservée à l'écoulement est sensiblement la même que celle d'où s'échappe l'oxygène, soit 100 $cm^2$ environ.

Chacun des canaux de section égale à 1 $mm^2$ canalise la charge de façon sensiblement parallèle à l'axe du réacteur et la distribue de manière uniforme dans l'espace confiné entre les conduits 3, 4 de distribution d'oxygène et le monolithe 7 et un second monolithe 8 situé sous ces conduits.

Cet espace est sensiblement égal à 1 mm. Dans ces conditions, on minimise les phénomènes de rétromélange.

Les fluides gazeux s'écoulent entre les conduits poreux.

De l'autre côté du distributeur en oxygène, se trouve une seconde capacité 28 comprenant le monolithe 8 qui comporte une pluralité de canaux 13 en carbure de silicium parallèles juxtaposés et parallèles également à l'axe du réacteur, le premier et le second monolithes étant perpendiculaires au plan radial contenant les conduits poreux d'alimentation en oxygène.

Leur section individuelle de forme carrée est de 1 mm² environ, la longueur de chaque canal est d'environ 60 cm.

Ces canaux sont d'une part destinés à canaliser les produits de réaction jusqu'à une ligne d'évacuation de produits 9 et d'autre part, en raison de leur faible largeur et par effet de paroi, à "coincer la flamme", ce qui permet à la réaction de se poursuivre sans explosion.

De manière pratique, il est possible de disposer de deux demi-coquilles de monolithe que l'on creuse de part et d'autre de la zone de distribution en oxygène de telle sorte qu'on aménage un ensemble de cavités 15 (Figs 6 et 6A) uniformes, parallèles entre elles et aux conduits poreux et perpendiculaires aux canaux 13 des monolithes 7 et 8, cavités dont les dimensions sont celles correspondant à la distance de coincement.

On assemble ensuite les demi-coquilles et l'ensemble (monolithes et distributeurs d'oxygène) est maintenu grâce par exemple à des cales 14 en matière céramique dans une virole en acier 10, protégé de manière conventionnelle par des couches 11 de béton réfractaire et/ou de béton ou de briques réfractaires isolantes suivant les règles de l'art bien connues quand on opère à haute température et sous pression.

Grâce à cette disposition et aux matériaux utilisés, on parvient à réaliser des réactions d'oxydation à des températures très élevées de l'ordre de 1.300°C, sans dépôt de carbone et avec des temps de séjour dans le réacteur n'excédant pas 1.000 ms.

On introduit par exemple, par la ligne 6 une charge oxydable comprenant du méthane et de la vapeur d'eau préchauffée à environ 450°C, dans un rapport molaire $H_2O/CH_4$ égal à environ 0,8. On introduit 0,55 moles d'oxygène par la ligne 12 à une température d'environ 150°C.

Les rapports molaires $O_2/CH_4$ que l'on préconise, par exemple 0,5 à 0,75 et dans cet exemple 0,55, peuvent permettre d'obtenir un gaz de synthèse dont le rapport molaire $H_2/CO$ est par exemple proche de 2, avec une température de sortie des gaz d'environ 1.080°C, le réacteur étant à une pression d'environ 20 bar.

Il est bien évident que le niveau de l'arrivée de la charge et le niveau de soutirage des produits réactionnels sont indifféremment choisis en position haute ou basse sur le réacteur.

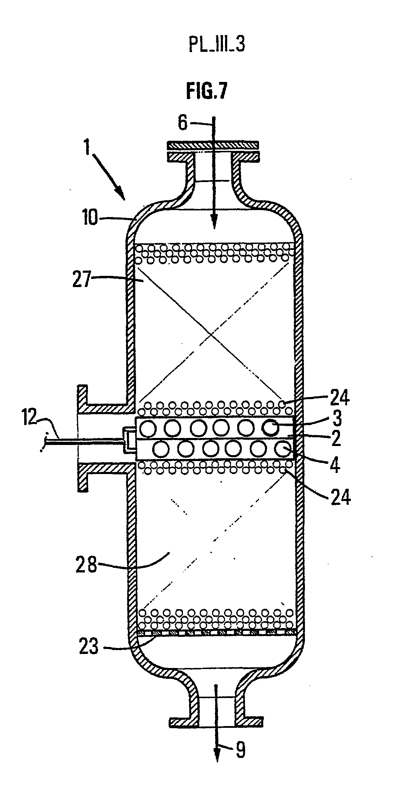

Selon un autre mode de réalisation illustré par la figure 7, le réacteur 1, tel que décrit dans la figure 1, contient une première capacité 27 et une seconde capacité 28 disposées de part et d'autre des distributeurs d'oxygène 2 et 20 de longueur sensiblement égale au diamètre du réacteur, soit 10 cm.

Les capacités sont dans ce cas remplies de billes 24 en carbure de silicium, de forme sphérique, ayant un diamètre de 0,5 mm choisi en fonction de la distance de coincement de la flamme.

Les billes adhèrent aux distributeurs d'oxygène de sorte que les espaces définis entre les parois des billes d'une part et entre le distributeur et le point de contact des billes d'autre part est au plus égal au rayon des billes (0,25 mm) qui devient la distance de coincement de la flamme.

La hauteur du garnissage 27 en amont des distributeurs 2 et 20 est par exemple de l'ordre de 0,40 m tandis que celle située en aval et qui constitue la zone réactionnelle proprement dite 28 est d'environ 1 m.

Une grille 23 retient le garnissage.

L'exemple qui suit est donné à titre illustratif.

Dans ce réacteur à billes fonctionnant sous 80 bars, on introduit par la ligne 6 une charge oxydable comprenant du méthane et de la vapeur d'eau préchauffés à 400°C dans un rapport molaire $H_2O/CH_4$ égal à 1,04.

On introduit par la ligne d'alimentation 12 de l'oxygène dans un rapport molaire $O_2/CH_4$ égal à 0,66 à une température d'environ 150°C.

Après réaction, les gaz sont à la température de 1.190°C et ont la composition suivante (% moles):
$CH_4$: 0,30%
CO: 19,8%
$H_3$: 44,7%
$CO_2$: 5,8%
$H_2O$: 29,3%

On ne sortira pas du cadre de la présente invention en construisant, par exemple, un réacteur comprenant, pour la première capacité, au moins un monolithe et un remplissage d'éléments particulaires et une seconde capacité comportant un garnissage d'éléments particulaires, ou inversement.

**Revendications**

1. Procédé d'oxydation d'une charge oxydable en phase gazeuse par un mélange de gaz contenant au moins un gaz oxydant dans lequel on recueille des produits réactionnels, caractérisé en ce que l'on met en contact ledit mélange de gaz et ladite charge oxydable dans une zone de mise en contact et de mélange située entre au moins une première zone parcourue par ladite charge et au moins une seconde zone parcourue par les produits réactionnels d'oxydation ainsi obtenus, lesdites première et seconde zones définissant une multiplicité d'espaces multidirectionnels présentant des passages ayant, suivant au moins une direction, une dimension au plus égale à 10 millimètres, cette dimension correspondant à la distance de coincement de la flamme pouvant résulter de l'oxydation de ladite charge, ladite zone de mise en contact comprenant une zone d'alimentation en mélange oxydant qui comporte une pluralité de conduits sensiblement parallèles à parois poreuses et qui est située à une distance de la première zone et de la seconde zone au plus égale à la distance de coincement.

2. Procédé selon la revendication 1 dans lequel on fait s'écouler ladite charge, les produits réactionnels et ledit mélange oxydant dans une première zone, une seconde zone et des conduits dont les parois sont en matière céramique, respectivement.

3. Réacteur pour la mise en oeuvre du procédé selon la revendication 1, comportant des moyens d'alimentation en gaz oxydant (12), des moyens d'alimentation en charge oxydable (6) et des moyens d'évacuation des produits réactionnels (9), caractérisé en ce qu'il comporte en combinaison:
— au moins un distributeur en gaz oxydant (2) relié d'une part aux moyens d'alimentation (12) en gaz oxydant et d'autre part à une pluralité de conduits sensiblement parallèles (3, 4) à parois poreuses en matière céramique,
— au moins une première capacité (27) comportant un garnissage en matière céramique qui définit une multiplicité d'espaces multidirectionnels présentant des passages ayant, suivant au moins une direction, une dimension au plus égale à 10 mm, cette dimension correspondant à la distance de coincement de la flamme pouvant résulter de l'oxydation de ladite charge, ladite première capacité étant reliée auxdits moyens d'alimentation en charge oxydable (6), et
— au moins une seconde capacité (28) comportant un garnissage en matière céramique qui définit une multiplicité d'espaces multidirectionnels présentant des passages ayant, suivant au moins une direction, une dimension au plus égale à 10 mm, cette dimension correspondant à la distance de coincement de la flamme pouvant résulter de l'oxydation de ladite charge, ladite seconde capacité étant reliée auxdits moyens d'évacuation (9), ladite première et ladite seconde capacités étant situées de part et d'autre desdits conduits poreux (3, 4) à une distance au plus égale à la distance de coincement.

4. Réacteur selon la revendication 3, caractérisé en ce que l'une au moins desdites capacités (27 ou 28) renferme le garnissage sous forme d'éléments particulaires de taille comprise entre 0,01 et 10 mm.

5. Réacteur selon la revendication 3, caractérisé en ce que l'une au moins desdites capacités (27 ou 28) comportant un garnissage, comprend au moins un monolithe (7 ou 8) qui comporte une pluralité de canaux (13) sensiblement parallèles juxtaposés, chacun desdits canaux (13) ayant une section comprise entre 0,0025 mm2 et 100 mm2.

6. Réacteur selon la revendication 3, caractérisé en ce que l'une au moins desdites capacités (27 ou 28) renferme le garnissage comprenant au moins un monolithe (7 ou 8) qui comporte une pluralité de canaux (13) sensiblement parallèles juxtaposés, au moins un des canaux comportant au moins en partie un remplissage sous forme d'éléments particulaires (24) de taille comprise entre 0,01 et 10 mm.

7. Réacteur selon l'une quelconque des revendications 3 à 6, caractérisé en ce que chacune desdites capacités (27, 28) présente en section par un plan, une surface d'écoulement de la charge et des produits réactionnels sensiblement égale à celle occupée par lesdits conduits poreux (3, 4).

8. Réacteur selon la revendication 7, caractérisé en ce que les surfaces d'écoulement de la première et de la seconde capacité (27, 28) sont sensiblement vis-à-vis de la surface d'écoulement du gaz oxydant des conduits (3, 4).

9. Réacteur selon l'une quelconque des revendications 3 à 8, caractérisé en ce qu'une partie au moins de la section du réacteur est entièrement couverte par lesdits conduits poreux.

10. Réacteur selon l'une quelconque des revendications 3 à 9, caractérisé en ce que les conduits poreux (3, 4) sont disposés en couches décalées non adjacentes, de part et d'autre du distributeur en gaz oxydant (2).

11. Réacteur selon l'une quelconque des revendications 3 à 9, caractérisé en ce qu'il comporte deux distributeurs (2, 20) en gaz oxydant, chacun d'eux étant relié à une pluralité de conduits à parois poreuses, sensiblement parallèles, non adjacents et définissant ainsi une première et une seconde couche de conduits, lesdits conduits de la première couche étant décalés par rapport à ceux de la seconde couche.

12. Réacteur selon l'une quelconque des revendications 3 à 11, caractérisé en ce que la largeur de chaque conduit poreux est comprise entre environ 5 mm et 50 mm.

13. Réacteur selon l'une quelconque des revendications 3 à 12, caractérisé en ce que l'écartement entre deux conduits poreux d'une même couche est au plus égal à la largeur dudit conduit poreux.

14. Réacteur selon l'une quelconque des revendications 3 à 13, caractérisé en ce que la porosité desdits conduits est choisie de telle sorte que la perte de charge en oxygène soit comprise entre 0,05 et 10 bars.

15. Réacteur selon l'une quelconque des revendications 3 à 12 et 14, dans lequel les conduits poreux sont adjacents, caractérisé en ce qu'au moins un d'entre eux est percé de part et d'autre d'un plan longitudinal d'une pluralité de trous de dimension comprise entre 0,05 et 10 mm.

16. Utilisation du réacteur selon l'une quelconque des revendications 3 à 15 notamment pour la synthèse de méthanol et d'alcools homologues supérieurs à partir d'oxydes de carbone et d'hydrogène.

**Patentansprüche**

1. Verfahren zur Oxidation einer oxidierbaren Charge in gasförmiger Phase durch ein wenigstens ein oxidierendes Gas enthaltendes Gasgemisch bei dem man Reaktionsprodukte sammelt, dadurch gekennzeichnet, daß man dieses Gasgemisch und diese oxidierbare Charge in einer Kontakt- und Mischzone kontaktiert, die zwischen wenigstens einer ersten Zone, die von dieser Charge durchströmt wird und wenigstens einer zweiten Zone angeordnet ist, die von den so erhaltenen Oxidationsreaktionsprodukten durchströmt wird, wobei diese ersten und zweiten Zonen eine Vielzahl von multidirektionellen Räumen bilden, welche Durchlässe aufweisen, die entsprechend wenigstens einer Richtung eine Abmessung haben, die höchstens gleich 10 Millimeter beträgt, wobei diese Abmessung dem Festkeilabstand der Flamme entspricht, die aus der Oxidation der Charge resultieren kann, wobei diese Kontaktzone eine Zone für die Speisung mit oxidierbarem Gemisch umfaßt, die eine Vielzahl von Leitungen aufweist, die im wesentlichen parallel zu porösen Wandungen sind und die unter einem Abstand zur ersten Zone und zur zweiten Zone angeordnet ist, der höchstens gleich der Festkeilentfernung ist.

2. Verfahren nach Anspruch 1, dadurch gekennzeichnet, daß man diese Charge, die Reaktionsprodukte sowie das oxiderende Gemisch in einer ersten Zone, einer zweiten Zone sowie Leitungen strömen läßt, deren Wandungen jeweils aus keramischem Material bestehen.

3. Reaktor zur Durchführung des Verfahrens nach Anspruch 1, mit Einrichtungen zur Speisung mit oxidierbarem Gas (12), Einrichtungen zum Speisen mittels oxidierbarer Charge (6) sowie Einrichtungen zum Abführen der Reaktionsprodukte (9), dadurch gekennzeichnet, daß er in Kombination umfaßt:

— wenigstens einen Verteiler für oxidierendes Gas (2), der einerseits mit den Speiseeinrichtungen (12) für oxidierendes Gas und andererseits mit einer Vielzahl von im wesentlichen parallelen Leitungen (3, 4) mit porösen Wandungen aus keramischem Material verbunden ist,

— wenigstens eine erste Kammer (27) mit einer Auskleidung aus keramischem Material, die eine Vielzahl von multidirektionellen Räumen definiert, welche Durchlässe aufweisen, die, längs wenigstens einer Richtung eine Abmessung von höchstens 10 mm haben, wobei diese Abmessung der Festkeilentfernung der Flamme entspricht, die aus der Oxidation dieser Charge resultieren kann, wobei die erste Kammer mit diesen Einrichtungen zur Speisung mit oxidierbarer Charge (6) verbunden ist, und

— wenigstens eine zweite Kammer (28) mit einer Auskleidung aus keramischem Material, die eine Vielzahl von multidirektionellen Räumen definiert, welche Durchlässe längs wenigstens einer Richtung, mit einer Abmessung von höchstens 10 mm bieten, wobei diese Abmessung der Festkeilentfernung der Flamme entspricht, die aus der Oxidation dieser Charge resultieren kann, wobei die zweite Kammer mit diesen Abführeinrichtungen (9) verbunden ist, wobei diese ersten und zweiten Kammern zu beiden Seiten dieser porösen Leitungen (3, 4) unter einem Abstand angeordnet sind, der höchstens gleich dem Festkeilabstand ist.

4. Reaktor nach Anspruch 3, dadurch gekennzeichnet, daß wenigstens eine dieser Kammern (27 oder 28) die Auskleidung in Form von besonderen Elementen einer Abmessung zwischen 0,01 und 10 mm umschließt.

5. Reaktor nach Anspruch 3, dadurch gekennzeichnet, daß wenigstens eine dieser Kammern (27 oder 28), die eine Auskleidung umfaßt, wenigstens ein monolithisches Gebilde (7 oder 8) umfaßt, welches über eine Vielzahl von im wesentlichen parallelen nebeneinander angeordneten Kanälen (13) verfügt, wobei jeder dieser Kanäle (13) einen Querschnitt zwischen 0,0025 mm$^2$ und 100 mm$^2$ aufweist.

6. Reaktor nach Anspruch 3, dadurch gekennzeichnet, daß wenigstens eine dieser Kammern (27 oder 28) die Auskleidung umschließt, welche wenigstens ein monolithisches Gebilde (7 oder 8) umfaßt, welches eine Vielzahl von im wesentlichen nebeneinander angeordneten parallelen Kanälen (13) aufweist und wobei wenigstens einer der Kanäle wenigstens zum Teil eine Füllung in Form von besonderen Elementen (24) einer Abmessung zwischen 0,01 une 10 mm aufweist.

7. Reaktor nach einem der Ansprüche 3 bis 6, dadurch gekennzeichnet, daß jede dieser Kammern (27, 28) im Schnitt längs einer Ebene, insbesondere einer planen Ebene, eine Strömungsfläche für die Charge und die Reaktionsprodukte aufweist, die im wesentlichen gleich der durch diese porösen Leitungen (3, 4) eingenommenen ist.

8. Reaktor nach Anspruch 7, dadurch gekennzeichnet, daß die Strömungsflächen der ersten und der zweiten Kammer (27, 28) im wesentlichen gegenüber der Strömungsfläche des oxidierenden Gases der Leitungen (3, 4) stehen.

9. Reaktor nach einem der Ansprüche 3 bis 8, dadurch gekennzeichnet, daß wenigstens ein Teil des Reaktorquerschnitts völlig durch diese porösen Leitungen überdeckt ist.

10. Reaktor nach einem der Ansprüche 3 bis 9, dadurch gekennzeichnet, daß die porösen Leitungen (3, 4) in nicht benachbarten versetzten Schichten zu beiden Seiten des Verteilers für oxidierendes Gas (2) angeordnet sind.

11. Reaktor nach einem der Ansprüche 3 bis 9, dadurch gekennzeichnet, daß dieser zwei Verteiler (2, 20) für oxidierendes Gas umfaßt, von denen jeder mit einer Vielzahl von Leitungen mit porösen Wandungen verbunden ist, die im wesentlichen parallel verlaufen, nicht aneinander angrenzen und so eine erste und eine zweite Schicht aus solchen Leitungen bilden, wobei die Leitungen der ersten Schicht bezogen auf die der zweiten Schicht versetzt sind.

12. Reaktor nach einem der Ansprüche 3 bis 11, dadurch gekennzeichnet, daß die Breite jeder porösen Leitung zwischen etwa 5 mm und etwa 50 mm beträgt.

13. Reaktor nach einem der Ansprüche 3 bis 12, dadurch gekennzeichnet, daß der Abstand zwischen zwei porösen Leitungen ein und der gleichen Schicht wenigstens gleich der Breite dieser porösen Leitung ist.

14. Reaktor nach einem der Ansprüche 3 bis 13, dadurch gekennzeichnet, daß die Porosität dieser Leitungen derart gewählt ist, daß der Sauerstoffdruckverlust zwischen 0,05 und 10 Bar liegt.

15. Reaktor nach einem der Ansprüche 3 bis 12 und 14, wobei die porösen Leitungen einander benachbart sind, dadurch gekennzeichnet, daß wenigstens eine hiervon zu beiden Seiten einer Längsebene von einer Vielzahl von Löchern einer Abmessung durchbohrt ist, die zwischen 0,05 und 10 mm beträgt.

16. Verwendung des Reaktors nach einem der Ansprüche 3 bis 15, insbesondere für die Synthese von Methanol und höheren homologen Alkoholen ausgehend von Oxiden des Kohlenstoffs und Wasserstoffs.

## Claims

1. A method for oxidizing a charge that can be oxidized in gaseous phase by a mixture of gases containing at least one oxidizing gas in which the reaction products are collected, characterised in that said mixture of gases and said oxidizable charge are brought into contact in a contact and mixing zone located between at least one first zone passed through by said charge and at least one second zone passed through by the oxidation reaction products thus obtained, said first and second zones defining a multiplicity of multi-directional spaces exhibiting passages having one dimension in at least one direction at most equal to 10 millimetres, this dimension corresponding to the arresting distance of the flame which could result from oxidation of said charge, said contact zone comprising a zone for supplying oxidizing mixture which comprises a plurality of substantially parallel ducts with porous walls and is located a distance from the first zone and the second zone at most equal to the arresting distance.

2. A method as in claim 1 in which said charge, the reaction products and said oxidizing mixture are passed respectively through a first zone, a second zone and ducts the walls of which are made of ceramic material.

3. A reactor for implementing the method as in claim 1, comprising means for supplying oxidizing gas (12), means for supplying oxidizable charge (6) and means for discharging the reaction products (9), characterised in that it comprises in combination:
   – at least one distributor for oxidizing gas (2) connected firstly to the means for supplying oxidizing gas (12) and secondly to a plurality of substantially parallel ducts (3, 4) with porous walls made of ceramic material;
   – at least one first capacity (27) comprising a lining made of ceramic material which defines a multiplicity of multi-directional spaces exhibiting passages having one dimension in at least one direction at most equal to 10 mm, this dimension corresponding to the arresting distance of the flame which could result from the oxidation of said charge, said first capacity being connected to said means for supplying oxidizable charge (6), and
   – at least one second capacity (28) comprising a lining made of ceramic material which defines a multiplicity of multi-directional spaces exhibiting passages having one dimension in at least one direction at most equal to 10 mm, this dimension corresponding to the arresting distance of the flame which could result from the oxidation of said charge, said second capacity being connected to said discharge means (9), said first and said second capacities being located on either side of said porous ducts (3, 4) at a distance at most equal to the arresting distance.

4. A reactor as in claim 3, characterised in that at least one of said capacities (27 or 28) incorporates the lining in the form of particulate elements of a size of between 0.01 and 10 mm.

5. A reactor as in claim 3, characterised in that at least one of said capacties (27 or 28) having a lining comprises at least one monolith (7 or 8) which comprises a plurality of substantially parallel juxtaposed channels (13), each of said channels (13) having a section of between 0.0025 $mm^2$ and 100 $mm^2$.

6. A reactor as in claim 3, characterised in that at least one of said capacities (27 or 28) incorporates the lining comprising at least one monolith (7 or 8) which comprises a plurality of substantially parallel juxtaposed channels (13), at least one of the channels being at least partially filled with particulate elements (24) of a size of between 0.01 and 10 mm.

7. A reactor as in any one of claims 3 to 6, characterised in that in section through a plane each of said capacities (27, 28) exhibits a flow surface for the charge and the reaction products substantially equal to that taken up by said porous ducts (3, 4).

8. A reactor as in claim 7, characterised in that the flow surfaces of the first and the second capacity (27, 28) are substantially opposite the flow surface of the ducts (3, 4) for the oxidizing gas.

9. A reactor as in any one of claims 3 to 8, characterised in that at least part of the section of the reactor is entirely covered by said porous ducts.

10. A reactor as in any one of claims 3 to 9, characterised in that the porous ducts (3, 4) are disposed in staggered non-adjacent layers on either side of the distributor for oxidizing gas (2).

11. A reactor as in any one of claims 3 to 9, characterised in that it comprises two oxidizing gas distributors (2, 20), each of them being connected to a plurality of substantially parallel non-adjacent ducts with porous walls, thus defining a first and a second layer of ducts, said ducts of the first layer being staggered in relation to those of the second layer.

12. A reactor as in any one of claims 3 to 11, characterised in that the width of each porous duct is between about 5 mm and 50 mm.

13. A reactor as in any one of claims 3 to 12, characterised in that the distance between two porous ducts of the same layer is at most equal to the width of said porous duct.

14. A reactor as in any one of claims 3 to 13, characterised in that the porosity of said ducts is preferably chosen so that the loss of head of oxygen is between 0.05 and 10 bars.

15. A reactor as in any one of claims 3 to 12 and 14, in which the porous ducts are adjacent, characterised in that at least one of them is drilled on either side of a longitudinal plane with a plurality of holes of a dimension of between 0.05 and 10 mm.

16. Use of the reactor as in any one of claims 3 to 15 in particular for synthesis of methanol and higher homologous alcohols from oxides of carbon and hydrogen.

EP 0 221 813 B1

PL.I.3

FIG.1

FIG.2

FIG.2A

FIG.3

FIG.3A

FIG.4A

FIG.4B

FIG.4C

PL_II_3

**FIG.5**

13

**FIG.5A**

13 13

(7 7)
(8 8)

**FIG.6**

7

3

14

15

4

8

**FIG.6A**

7

2

3

14

12

15

20

4

8

PL_III_3

**FIG.7**